# EUROPEAN PATENT SPECIFICATION

(11) **EP 2 716 235 B1**
(45) Date of publication and mention of the grant of the patent: **07.12.2016**
(21) Application number: 12187056.2
(22) Date of filing: 02.10.2012
(51) Int. Cl.: A61B 17/06

(54) **Suture package and method for winding a suture in a suture package**
Fadenverpackung und Verfahren zum Wickeln eines Fadens in einer Fadenverpackung
Emballage pour fils de suture et procédé d'enroulement de fil de suture dans un emballage de suture

(43) Date of publication of application: 09.04.2014
(73) Proprietor: B. Braun Surgical, S.A., 08191 Rubi (Barcelona) (ES)
(72) Inventor: Aranda García, Jose Antonio, 08226 Terrassa (ES); Afonso Sanmarti, Olga, 08202 Sabadell (ES)
(74) Representative: Patentanwälte Ruff, Wilhelm, Beier, Dauster & Partner mbB

(56) References cited:
- EP-A1- 0 717 958
- EP-A1- 1 475 046
- US-A1- 2003 010 655
- US-A1- 2004 177 594

## Description

### Technical Field

The invention relates to a suture package and a method for winding a suture in a suture package.

### Background of the Invention

It is known to retain surgical sutures having needles affixed at one or both ends in suture packages.

For example, EP 2 415 406 A1 discloses a suture package comprising a base member and a cover member, wherein the cover member is provided with an inner track wall and with an upper wall extending radially out from the inner track wall at an upper end thereof. The base member is formed as an oval tray and provided with an outside wall. The outside wall rises from a flat section of the base member and extends about the periphery of the base member. The outside wall is designed having a predetermined height such that an upper rim of the outside wall engages the cover member. Thus, when the base member and the cover member are coupled, the outer circumference of the cover is joined with the upper rim of the outside wall to form a closed suture package with a suture channel provided between the upper wall and the base member. For assembling the suture package, the cover member is positioned with its back side facing upward, a suture is wound onto the cover member, and then the base member is secured to the cover member. The inner track wall and the cover member are provided with openings to permit a withdrawal or removal of the suture from the package.

EP 1 214 912 A2 discloses a suture package comprising a base member having an outer track wall projecting from the base member about the periphery of the base member, and a top member having an inner track wall. When joining the base member and the top member, the inner track wall is arranged spaced away from the outer track wall to form a suture channel between the inner and the outer track wall. The top member further has a plurality of doors extending radially out from the inner track wall at an upper end thereof over the suture channel, wherein the doors are connected to the inner track wall via hinges.

EP 0 717 958 A1 shows a suture package comprising a circular plate forming an open channel for receiving a suture, which is closed after the suture is loaded into the channel by means of a flange member.

EP 1 475 046 A1 shows a suture package comprising a winding body having a surrounding winding channel for receiving the suture. The winding body is a one-pieced plastic member and one of the side walls of the winding channel is pivotable to close the winding channel.

US 2004/0177594 A1 discloses a machine and a process for packaging armed sutures into tray packages, the tray packages comprising a base member and top member having flaps 80. For packaging armed sutures into the tray packages, the flaps are pivotally raised upward by a winding stylus about a hinge point.
US 2003/0010655 A1 discloses a suture package comprising a base member having top and bottom surfaces. A needle park bridge is formed on the top surface and is sized and shaped to receive a needle of a suture. A suture channel is formed on the bottom surface and is sized and shaped to receive a suture. A suture channel cover is attached to the bottom surface, which suture channel cover has a plurality of cover door members.

### Summary of the Invention

It is the object of the present invention to provide a suture package in which the suture is retained readily accessible to permit a fast withdrawal or removal, wherein the suture may be packed in the package using a high-speed packaging machine.

It is further the object of the present invention to provide a method for packing a suture in a package at high-speed.

This and other objects are solved by a suture package and a method for winding a suture in a suture package with the features of claims 1 and 8.

According to a first aspect of the invention, a suture package comprising a tray-shaped base member with a central portion and with a peripheral wall portion and a cover member configured to attach to the base member with an inner track wall and with an upper wall extending radially out from the inner track wall at an upper end thereof is provided, wherein a suture channel is formed between the upper wall and the peripheral wall portion, wherein the peripheral wall portion is at least partly resiliently deformable to spread the peripheral wall portion away from the upper wall, wherein the peripheral wall portion comprises a plurality of lip portions distributedly provided over the circumference of the central portion and a first hinge for pivotably supporting the lip portions, and wherein the lip portions are connected via second hinges to form a continuous loop.

The term "resiliently deformable element" as used throughout this specification and in the claims means that the element has an appropriate resilience and deformability due to material properties and/or its geometric shape, so that the element is deformable when a force is applied to at least a portion of the element, but returns at least approximately to an initial shape when the applied force is removed.

The base member and the cover member in one embodiment are made of different materials. In particular, in one embodiment, the cover member is made of a stiff material to provide a sufficient rigidity to the suture package, whereas the base member is made of a resiliently deformable material permitting a deformation for accessing the channel. In one embodiment, the cover member is made of polyethylene (PE). The base member in one embodiment is made of polypropylene (PP).

The base member preferably is formed as an oval tray, having a needle holder provided at the central portion.

In one embodiment, an outer edge of the peripheral wall portion abuts the back surface of the upper wall to provide a closed suture channel. Upon a deformation of the peripheral wall portion, the suture channel becomes accessible for winding the suture to the package. When the force is removed, the suture package returns to its initial state, wherein in some embodiments the suture channel is closed. For a removal of the suture or withdrawal of the suture, in preferred embodiments a pulling force is applied to the suture and the suture slides inside the channel.

A hinge axis extends in preferred embodiments at least partly along the suture channel. By bending or pivoting the at least one lip portion relative to the central portion, the peripheral wall portion is at least partly spread away from the upper wall.
The lip portions each radially extend from the central portion, wherein an outer edge of the peripheral wall portion is formed by the outer ends of the lip portions. The lip portions are connected via hinges to form a continuous loop. Thereby, each lip portion or a number of adjacent lip portions may be deformed successively for accessing the channel.

According to an embodiment, the central portion and the at least one lip portion are manufactured as an integral component by using a flexure bearing, in particular a living hinge as the first hinge. When a force is applied, the lip portion is permitted to pivot at the living hinge or a similar flexure bearing about the hinge axis away from the upper wall. After the applied force is removed, the lip portion returns to the initial state in which for example its outer edge abuts the back surface of the upper wall.
In one embodiment, the upper wall is provided with at least one lip opening pin receiving hole. The lip opening pin receiving hole permits a force to be applied to the lip portions by inserting a pin in the lip opening pin receiving hole.

In one embodiment, the peripheral wall portion, more particular the lip portions are angulated with respect to the upper wall in an undeformed state, wherein an outer end of the peripheral wall portion abuts the back surface of the upper wall. In addition or as an alternative, in preferred embodiments, an outer track wall projecting towards the upper wall is provided at the outer end of the peripheral wall portion. Preferably, an upper rim of the outer track wall abuts the back surface of the upper wall. In preferred embodiments, the upper rim of the outer track wall abuts the periphery of the back surface of the upper wall.

According to another embodiment, the outer track wall and the upper wall are provided with interlocking protrusions. Preferably, the outer track wall and the upper wall are provided with teeth and ridges. The teeth and ridges allow an interlocking of the outer track wall and the upper wall for coupling the base member and the cover member in an undeformed state. In one embodiment, the teeth each have a sloped edge at a rear side in a direction of winding. The term "direction of winding" as used throughout this specification and in the claims means the movement direction of the suture along the channel for winding the suture. The terms "front side of the teeth" and "rear side of the teeth" as used throughout this specification and in the claims means the side of the teeth facing in the direction of winding or in the opposite direction, respectively. The upper rim of the outer track wall rests unsecured on the back surface, wherein the base member and the upper wall can be moved apart for providing a gap permitting an access to the suture. When winding the suture, the suture may slide along an upper rim of the outer track wall, wherein the teeth having a sloped edge support the movement in a direction of winding. In one embodiment, the suture is wound using a thread winding finger, wherein a direct contact between the suture and the outer track wall is avoided. As an alternative or in addition, a material of the base member is chosen such that frictional forces between the suture and the teeth during winding or unwinding are minimized. In other embodiments, a coating is provided at least in the area of the teeth. The coating is suitably chosen by the person skilled in the art to minimize frictional effects between the teeth and the suture and/or the thread winding finger.

According to a second aspect, a method for winding a suture in a suture package is provided, the suture package comprising a tray-shaped base member with a central portion and with a peripheral wall portion and a cover member configured to attach to the base member with an inner track wall and with an upper wall extending radially out from the inner track wall at an upper end thereof, wherein a suture channel is formed between the upper wall and the peripheral wall portion, wherein the peripheral wall portion comprises a plurality of lip portions distributedly provided over the circumference of the central portion and a first hinge for pivotably supporting the lip portions, and wherein the lip portions are connected via second hinges to form a continuous loop, the method comprising the step of resiliently deforming at least partly the peripheral wall portion by pivoting the lip portions about a hinge axis to spread the peripheral wall portion away from the upper wall.

By spreading the peripheral wall portion away from the upper wall a gap between the outer edge of the peripheral wall portion and the back surface of the upper wall is provided or increased, enhancing an accessibility to the suture channel for winding the suture. A small force is sufficient for providing or increasing the gap.

In one embodiment, a recess is provided in the upper wall and/or in the inner track wall for allowing the suture to enter the channel. For a removal of the suture, a pulling force is applied to the suture and the suture is removed via the recess. In one embodiment, when winding the suture a force is applied via the recess, for spreading the peripheral wall portion away from the upper wall.

In other embodiments, the upper wall of the cover member is provided with at least one lip opening pin receiving hole, wherein the method comprises the step of inserting at least one lip opening pin into the at least one lip opening pin receiving hole to spread the peripheral wall portion at least partly away from the upper wall.

In one embodiment, lip opening pin receiving holes are distributedly provided over the entire circumference, wherein the lip opening pin or a plurality of lip opening pins is successively inserted in sequentially arranged lip opening pin receiving holes. In a preferred embodiment, two lip opening pin receiving holes are provided. Further, in preferred embodiments, a thread winding finger is inserted into a gap between the peripheral wall portion and the upper wall. The thread winding finger is moved with respect to the suture package along the suture channel, wherein parts of the peripheral wall portion, in particular sequentially arranged lip portions are successively deformed.

In one embodiment, the suture is moved with or - less preferred - without a thread winding finger in loops around the suture package for winding the suture in the suture package. According to preferred embodiments, the suture package is rotated in a plane parallel to the base member for winding the suture onto the suture package.

### Brief Description of the Drawings

In the following, an embodiment of the invention will be described in detail with reference to the drawings. Throughout the drawings, the same elements will be denoted by the same reference numerals.

In the drawings:
- Fig. 1: shows a schematic top view of an embodiment of a suture package;
- Fig. 2: shows a schematic cross-sectional view along line II-II of Fig. 1;
- Fig. 3: shows a schematic cross-sectional view along line III-III of Fig. 1;
- Fig. 4: shows a detail IV of Fig. 3;
- Fig. 5: shows a schematic top view of a base member of the suture package of Fig. 1;
- Fig. 6: shows a schematic bottom view of a cover member of the suture package of Fig. 1;
- Figs. 7 - 10: show a top view of an embodiment of the suture package of Fig. 1 during winding of a suture.

### Detailed Description of an Embodiment

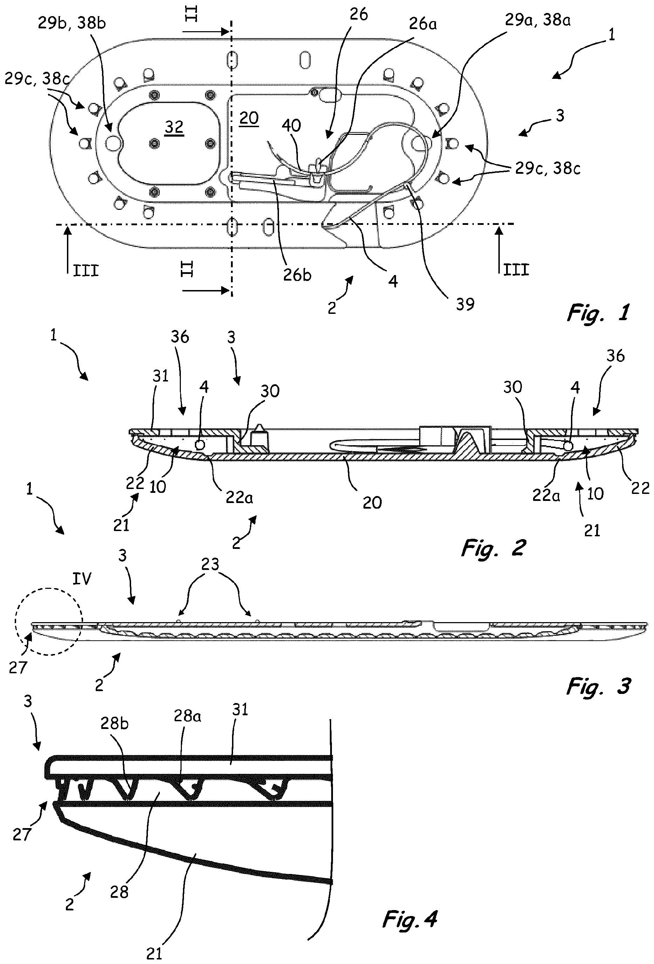

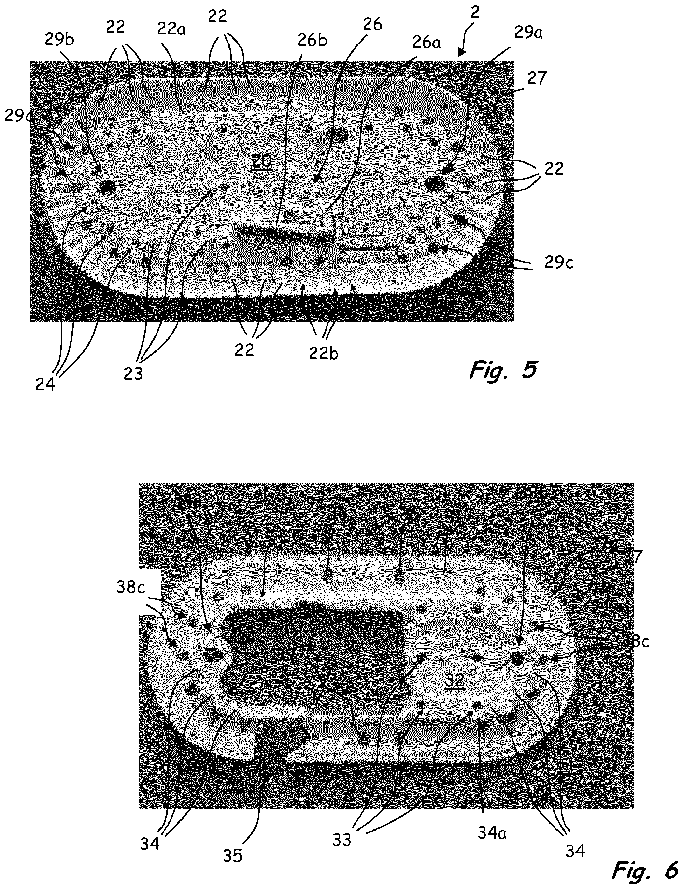

Figs. 1 to 4 schematically show an embodiment of a suture package 1 comprising a tray-shaped base member 2 and a cover member 3 configured to attach to the base member 2. More particular, Fig. 1 shows a top view of the suture package 1, Figs. 2 and 3 show cross-sectional views along lines II-II and III-III of Fig. 1, respectively, and Fig. 4 is a detail IV of Fig. 3. Fig. 5 shows a top view of the base member 2. Fig. 6 shows a bottom view of the cover member 3.

The base member 2 has a flat central portion 20 and a peripheral wall portion 21. The cover member 3 has an inner track wall 30 and an upper wall 31 extending radially out from the inner track wall 30 at an upper end thereof. The cover member 3 is configured to attach to the base member 2, wherein on the outer circumference of the suture package 1 a groove-shaped channel 10 is formed between the upper wall 31 and the peripheral wall portion 21 for receiving one or more windings of one or more sutures 4.

In the depicted embodiment, the base member 2 and the cover member 3 are approximately oval. However, although an oval shape of the suture package 1 is advantageous, the suture package 1 and, hence, the base member 2 and the cover member 3 may be configured with a different shape, including a polygonal shape, a circular shape and combinations thereof. Further, in the embodiment shown, the base member 2 and the cover member 3 are at least approximately of the same size.

The inner track wall 30 of the cover member 3 forms a core for a reel around which core the suture 4 is looped. In the depicted embodiment, the cover member 3 further comprises a planar top section 32, to which for example a label (not shown) may be attached. The planar top section 32 extends to the inside from the inner track wall 30 at a lower end thereof. When assembled, the lower end of the inner track wall 30 and the planar top section 32 abut to the central portion 20 of the base member 2.

The peripheral wall portion 21 comprises a plurality of lip portions 22 connected to the central portion 20 via hinges 22a. The lip portions 22 are distributedly provided over the circumference of the central portion 20. A hinge axis of the hinges 22 extends along the suture channel 10 in parallel to the inner track wall 30. In the embodiment shown, the central portion 20 and the lip portions 22 are manufactured as an integral component by using a living hinge as the hinges 22. In the embodiment shown, the plurality of lip portions 22 are formed integrally, wherein the lip portions 22 are connected via hinges, in particular living hinges 22b, to form a continuous loop allowing selected lip portions 22 to deform. In the undeformed or initial state, the lip portions 22 are angulated about the hinges 22 with respect to the central portion 20 and extend towards the cover member 3 for closing the suture channel 10. For accessing the suture channel 10, in particular during winding of the suture 4 to the suture package 1, the lip portions 22 are successively pivoted about the hinges 22a as will be described in more detail below.

For attaching the cover member 3 to the base member 2, locating pins 23 are provided at the base member 20, which are extending upwardly from the central portion 20 of the base member 2. The locating pins 23 are received by holes 33 provided at the back surface in the top section 32 of the cover member 3. Further, locating pins 34 are provided at the cover member 3, which are extending downwardly from the back surface the cover member 3 in a region close to and along the inner track wall 30. The locating pins 34 are received by holes 24 provided at the base member 2. The locating pins 23, 34 are preferably circular in cross-section, but may have other geometrical cross-sections including oval, square, polygonal, and the like and equivalents thereof. Although not preferred, locating pins 23, 34 may be replaced by other conventional fastening devices including locking pins, screws, etc. For fixing the cover member 3 to the base member 2 the locating pins 23, 34 are aligned with and inserted into the respective holes 33, 24. Then, ends of the locating pins 34 are spread by using conventional techniques such as welding, in particular ultrasonic welding, heating, and the like such that the cover member 3 is firmly affixed to the base member 2. In preferred embodiment, the ends of the locating pins 23 are not spread. In this case, the ends stick out from the cover member 3 as shown in Fig. 2 and may be used for attaching a label or the like (not shown) to the assembled suture package 1. Between the locating pins 34 of the cover member 3, guiding pins 34a are provided that hinder a suture 4 from entering a slit remaining between the inner track wall 30 and the base member 2 when the cover member 3 is affixed to the base member 2. In the depicted embodiment a single-armed suture 4 is provided having one needle 40 affixed to one end. In other embodiments, double-armed surgical sutures are retained in the suture package 1, wherein needles are affixed to both ends of the suture. The needle 40 is held in a needle holder 26 provided on the base member 2. In the present embodiment, one needle holder 26 is provided for the one needle. In other embodiments, two or more needle holders are provided for holding needles affixed to both ends of the sutures. The needle holder 26 is designed as a spring-loaded tension-mounting device, with a fixed bearing 26a and with an elongate spring arm 26b. The fixed bearing 26a is formed in one piece with and projecting from the central portion 20 of the base member 2. The spring arm 26b is connected fixedly at one end to the base member 3 and pivotable against the restoring forces parallel to the plane of the base member 2. Alternative configurations of needle holders are described for example in EP 2 153 780 A1.

The upper wall 31 of the cover member 3 and the inner track wall 30 are provided with a recess 35, through which the suture 4 enters into the suture channel 10. In the depicted embodiment, a V-shaped entry opening is formed at the upper wall 31 for guiding the suture 4 into the suture channel 10. Further, a guiding means 39 is provided extending from the inner track wall 30 towards the inside of the cover member 3. The suture 4 is guided by the guiding means 39 provided on the cover member 3 towards the recess 35. For a removal of the suture 4, a user grabs the needle 40 and applies a pulling force. The suture 4 slides in the channel 10 and is removed via the recess 39.

As described above, for accessing the suture channel 10, in particular during winding of the suture 4 to the suture package 1, the lip portions 22 are successively pivoted about the hinge axis. The upper wall 31 is provided with four lip opening pin receiving holes 36. An area at the recess 35 is referred to as entry area. In other embodiments, the lip opening pin receiving holes 36 provided opposite the entry area are omitted. A lip opening pin is inserted through the lip opening pin receiving holes 36 for applying a force to the lip portions 22. By applying the force, the lip portions 22 are pivoted and forced apart from the upper wall 31 for accessing the channel 10 during winding of the suture 4.

After withdrawal of the lip opening pin, the lip portions 22 return to the initial position due to material restoring forces. In the depicted embodiment, in the initial position shown in Figs. 1 to 4, the lip portions 22 are angulated and biased by the restoring forces against the upper wall 31 for closing the channel 10.

In addition, an outer track wall 27 is provided at the outer end of the peripheral wall portion 21, wherein the lip portions 22 are connected via the outer track wall 27. The upper rim, or short rim, of the outer track wall 27 abuts to the back surface of the cover member 3, more particular abuts to the back surface of the upper wall 31 at a periphery thereof. In the depicted embodiment, a peripheral groove 37 is provided at the periphery of the upper wall 31, wherein the outer track wall 27 abuts the back surface at the area of the peripheral groove 37.

As best seen in the detail of Fig. 4, teeth 28 are provided at a rim or upper rim of the outer track wall 27 projecting towards the cover member 3. In the depicted embodiment, the teeth 28 are asymmetrical and provided with a gently sloped edge 28a and a steeply sloped edge 28b. The gently sloped edges 28a are provided at a rear side of the teeth 28 seen in direction of winding. Hence, when winding the suture 4 on the suture package 1, the suture 4 (and/or a finger forcing the channel 10 into an open position) slides over the gently sloped edges 28a, wherein a movement in the opposite direction is hindered by the steeply sloped edges 28b. In one embodiment, a material of the base member 3 is chosen such that frictional forces between the suture 4 and the teeth 28 during winding or unwinding are minimized. In other embodiments, a coating is provided at least in the area of the teeth 28.

Between the teeth 28, grooves are formed. At the back surface of the upper wall 31, more particular in the area of the peripheral groove 37, ridges 37a are provided. When closing the channel 10, the teeth 26 and the ridges 37a interlock. In the depicted embodiment, the ridges 37a are provided in the region of the peripheral groove 37 and upper ends of the ridges 37a are flush with a back surface of the cover member 3. At the area 27a of the outer track wall 27 that comes to rest in the region of the recess 35 of the cover member 3 when attaching the cover member 3 to the base member 2, no teeth 28 are formed. Thereby, an upstanding section of the outer track wall 27 is provided for retaining the windings of the suture 4.

For an automatic winding, the base member 2 and the cover member 3 are further provided with aligned winding holes 29a, 29b, 29c, 38a, 38b, 38c. The winding holes 29a, 29b, 38a, 38b engage with fixing pins (not shown) for fixing the suture package 1 to a winding machine. Winding pins (not shown) are inserted via the winding holes 29c, 38c, wherein the suture 4 is looped around an imaginary core formed by the winding pins at a distance to the inner track wall 30. After removal of the winding pins, the suture 4 is loosely wound retained, allowing for a quick withdrawal.

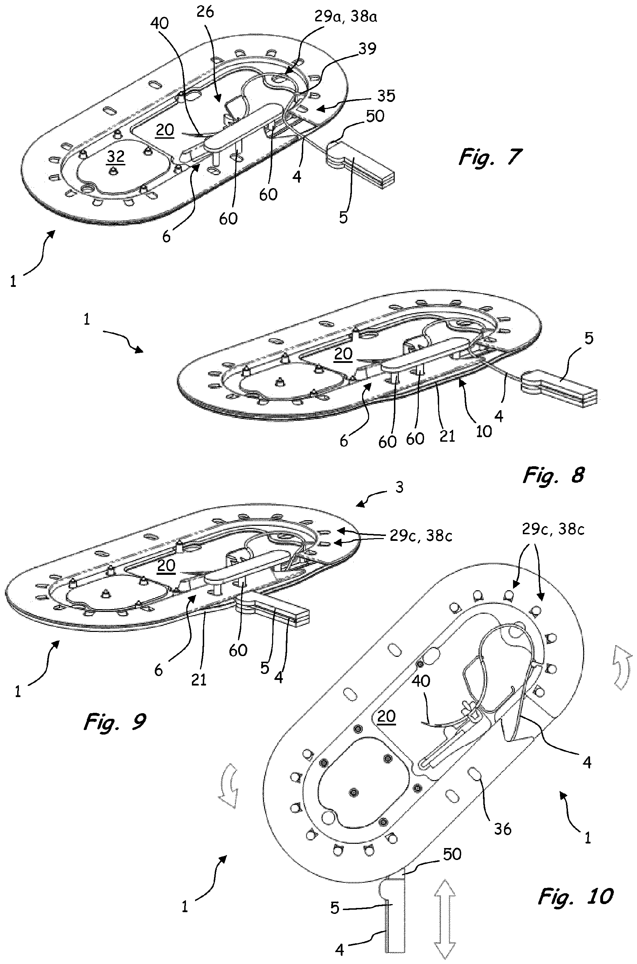

In the following, a method for winding the suture 4 in the suture package 1 according to Figs. 1 to 4 will be described with reference to Figs. 7 to 10. Throughout the drawings, the same or similar elements will be denoted by the same reference numerals.

As shown in Fig. 7, initially, the needle 40 of the suture 4 is affixed to the needle holder 26 and the suture 4 is gripped by a thread winding finger 5. The thread winding finger comprises a forwardly protruding tip 50 pointing towards the channel 10. The suture 4 extending from the needle 40 is looped around a fixing pin (not shown) inserted through the winding pin holes 28a, 38a. The suture 4 is guided by the guiding means 39 provided on the cover member 3 towards the recess 35.

An opening means 6, comprising three lip opening pins 60 is lowered towards the suture package 1 and the three lip opening pins 60 are inserted into the two lip opening pin receiving holes 36 provided at the entry area and the recess 35. The inserted lip opening pins 60 abut against the lip portions 22 of the base member 2 and force a part of the peripheral wall, more particular the lip portions 22 arranged in that area, away from the upper wall 31 of the cover member 3, as shown in Fig. 8. Thereby, the channel 10 of the suture package 1 is spread apart and the suture 4 may enter into the channel 10.

As shown in Fig. 9, the thread winding finger 5 is moved in radial direction towards the suture package 1 and enters with its tip 50 into the channel 10. After insertion of the tip 50 into the channel 10, the channel 10 is held open by means of the tip 50 and the lip opening means 6 may be removed.

After a removal of the lip opening means 6, the suture package 1 may be rotated for winding the suture 4 onto the suture package 1 as shown in Fig. 10. During a rotation of the suture package 1, the thread winding finger 5 slides along the lip portions 22. In particular, the tip 50 of the thread winding finger 5 slides over the gently sloped edges 28a (see Fig. 4), wherein a movement of the finger 5 in the opposite direction is hindered by the steeply sloped edges 28b. As schematically shown in Fig. 10, the thread winding finger 5 is moved along its axial direction during winding to ensure that contact is maintained between the suture package 1 and the thread winding finger 5.

When winding the suture 4 onto the suture package 1, a small gap is sufficient for allowing the suture 4 to enter into the channel 10. Therefore, a pivot angle to pivot the lip portions 22 may be chosen small for avoiding undue stresses. Nevertheless, the integrity of the sutures 4 during winding is ensured and a high-speed winding of the sutures 4 onto the suture package 1 is possible.

## Claims

1. Suture package comprising
- a tray-shaped base member (2) with a central portion (20) and with a peripheral wall portion (21) and
- a cover member (3) configured to attach to the base member (2) with an inner track wall (30) and with an upper wall (31) extending radially out from the inner track wall (30) at an upper end thereof,
wherein
- a suture channel (10) is formed between the upper wall (31) and the peripheral wall portion (21),
- the peripheral wall portion (21) is at least partly resiliently deformable to spread the peripheral wall portion (21) away from the upper wall (31), and
- the peripheral wall portion (21) comprises a plurality of lip portions (22) distributedly provided over the circumference of the central portion (20) and a first hinge (22a) for pivotably supporting the lip portions (22),
**characterized in that** the lip portions (22) are connected via second hinges (22b) to form a continuous loop.

2. Suture package of claim 1, **characterized in that** a flexure bearing, in particular a living hinge is provided as the first hinge (22a).

3. Suture package of claim 1 or 2, **characterized in that** the upper wall (31) of the cover member (3) is provided with at least one lip opening pin receiving hole (36).

4. Suture package of any of claim 1 to 3, **characterized in that** an outer track wall (27) projecting towards the upper wall (31) is provided at the outer end of the peripheral wall portion (21).

5. Suture package of claim 4, **characterized in that** an upper rim of the outer track wall (27) abuts the periphery of the back surface of the upper wall (31).

6. Suture package of claim 4 or 5, **characterized in that** the outer track wall (27) and the upper wall (31) are provided with interlocking protrusions.

7. Suture package of claim 6, **characterized in that** the outer track wall and the upper wall are provided with teeth (28) and ridges (37a).

8. Method for winding a suture (4) in a suture package (1), the suture package (1) comprising a tray-shaped base member (2) with a central portion (20) and with a peripheral wall portion (21) and a cover member (3) configured to attach to the base member (2) with an inner track wall (30) and with an upper wall (31) extending radially out from the inner track wall (30) at an upper end thereof, wherein a suture channel (10) is formed between the upper wall (31) and the peripheral wall portion (21), wherein the peripheral wall portion (21) comprises a plurality of lip portions (22) distributedly provided over the circumference of the central portion (20) and a first hinge (22a) for pivotably supporting the lip portions (22), and wherein the lip portions (22) are connected via second hinges (22b) to form a continuous loop, the method comprising resiliently deforming at least partly the peripheral wall portion (21) by pivoting the lip portions (22) about a hinge axis to spread the peripheral wall portion (21) at least partly away from the upper wall (31).

9. Method of claim 8, wherein the upper wall (31) of the cover member (3) is provided with at least one lip opening pin receiving hole (36), and wherein the method comprises inserting at least one lip opening pin (60) into the at least one lip opening pin receiving hole (36) to spread the peripheral wall portion (21) at least partly away from the upper wall (31).

10. Method of any of claims 8 or 9, wherein a thread winding finger (5) is inserted into a gap between the peripheral wall portion (21) and the upper wall (31).

11. Method of any of claim 8 to 10, wherein the suture package (1) is rotated in a plane parallel to the base member (3) for winding the suture (4) onto the suture package (1).

## Patentansprüche

1. Fadenverpackung, umfassend
- ein schalenförmiges Basiselement (2) mit einem Mittelabschnitt (20) und mit einem Umfangswandabschnitt (21) sowie
- ein Abdeckungselement (3), das zum Anbringen am Basiselement (2) ausgebildet ist, mit einer inneren Führungswand (30) und mit einer oberen Wand (31), die sich von der inneren Führungswand (30) ausgehend an deren oberem Ende radial erstreckt,
wobei
- ein Fadenkanal (10) zwischen der oberen Wand (31) und dem Umfangswandabschnitt (21) ausgebildet ist,
- der Umfangswandabschnitt (21) mindestens abschnittsweise nachgiebig verformbar ausgebildet ist, so dass der Umfangswandabschnitt (21) von der oberen Wand (31) weg gezogen wird, und
- der Umfangswandabschnitt (21) eine Mehrzahl von Lippenabschnitten (22), die über den Umfang des Mittelabschnitts (20) verteilt vorgesehen sind, und ein erstes Scharnier (22a) umfasst, mit dem die Lippenabschnitte (22) verschwenkbar gehaltert sind,
**dadurch gekennzeichnet, dass** die Lippenabschnitte (22) über zweite Scharniere (22b) verbunden sind, so dass eine Bandschleife ausgebildet ist.

2. Fadenverpackung nach Anspruch 1, **dadurch gekennzeichnet, dass** ein Festkörpergelenk, insbesondere ein Filmscharnier, als das erste Scharnier (22a) vorgesehen ist.

3. Fadenverpackung nach Anspruch 1 oder 2, **dadurch gekennzeichnet, dass** die obere Wand (31) des Abdeckungselements (3) mit mindestens einer Aufnahmeöffnung (36) für einen Lippenöffnungsstift versehen ist.

4. Fadenverpackung nach einem der Ansprüche 1 bis 3, **dadurch gekennzeichnet, dass** am äußeren Ende des Umfangswandabschnitts (21) eine zur oberen Wand (31) hin abragende äußere Führungswand (27) vorgesehen ist.

5. Fadenverpackung nach Anspruch 4, **dadurch gekennzeichnet, dass** ein oberer Rand der äußeren Führungswand (27) am Umfang der Rückseite der oberen Wand (31) anliegt.

6. Fadenverpackung nach Anspruch 4 oder 5, **dadurch gekennzeichnet, dass** die äußere Führungswand (27) und die obere Wand (31) mit aneinander verrastbaren Vorsprüngen versehen sind.

7. Fadenverpackung nach Anspruch 6, **dadurch gekennzeichnet, dass** die äußere Führungswand und die obere Wand mit Zähnen (28) und Rippen (37a) versehen sind.

8. Verfahren zum Wickeln eines Fadens (4) in einer Fadenverpackung (1), wobei die Fadenverpackung (1) umfasst: ein schalenförmiges Basiselement (2) mit einem Mittelabschnitt (20) und mit einem Umfangswandabschnitt (21) sowie ein Abdeckungselement (3), das zum Anbringen am Basiselement (2) ausgebildet ist, mit einer inneren Führungswand (30) und mit einer oberen Wand (31), die sich von der inneren Führungswand (30) ausgehend an deren oberem Ende radial erstreckt, wobei ein Fadenkanal (10) zwischen der oberen Wand (31) und dem Umfangswandabschnitt (21) ausgebildet ist, wobei der Umfangswandabschnitt (21) eine Mehrzahl von Lippenabschnitten (22) aufweist, die über den Umfang des Mittelabschnitts (20) verteilt vorgesehen sind, und ein erstes Scharnier (22a) umfasst, mit dem die Lippenabschnitte (22) verschwenkbar gehaltert sind, und wobei die Lippenabschnitte (22) über zweite Scharniere (22b) so verbunden sind, dass eine Bandschleife ausgebildet ist, und das Verfahren ein nachgiebiges Verformen des Umfangswandabschnitts (21) mindestens abschnittsweise durch Verschwenken der Lippenabschnitte (22) um eine Scharnierachse umfasst, so dass der Umfangswandabschnitt (21) mindestens abschnittsweise von der oberen Wand (31) weg gezogen wird.

9. Verfahren nach Anspruch 8, bei dem die obere Wand (31) des Abdeckungselements (3) mit mindestens einer Aufnahmeöffnung (36) für einen Lippenöffnungsstift versehen ist, und wobei das Verfahren ein Einsetzen mindestens eines Lippenöffnungsstifts (60) in die mindestens eine Aufnahmeöffnung (36) für einen Lippenöffnungsstift umfasst, so dass der Umfangswandabschnitt (21) mindestens abschnittsweise von der oberen Wand (31) weg gezogen wird.

10. Verfahren nach einem der Ansprüche 8 oder 9, bei dem ein Fadenwickelfinger (5) in einen Spalt zwischen dem Umfangswandabschnitt (21) und der oberen Wand (31) eingesetzt wird.

11. Verfahren nach einem der Ansprüche 8 bis 10, bei dem die Fadenverpackung (1) in einer Ebene parallel zum Basiselement (3) gedreht wird, um den Faden (4) auf die Fadenverpackung (1) zu wickeln.

## Revendications

1. Paquet de fil de suture comprenant :
- un élément de base en forme de plateau (2) ayant une partie centrale (20) et une partie de paroi périphérique (21) et
- un élément de couvercle (3) configuré de sorte à être fixé à l'élément de base (2) et ayant une paroi de piste interne (30) et une paroi supérieure (31) s'étendant radialement en dehors de la paroi de piste interne (30) au niveau d'une extrémité supérieure de celle-ci,
dans lequel
- un canal de fil de suture (10) est formé entre la paroi supérieure (31) et la partie de paroi périphérique (21),
- la partie de paroi périphérique (21) est au moins en partie déformable élastiquement pour écarter la partie de paroi périphérique (21) de la paroi supérieure (31), et
- la partie de paroi périphérique (21) comprend une pluralité de parties de lèvre (22) disposées de manière répartie sur la circonférence de la partie centrale (20) et une première articulation (22a) pour supporter de manière pivotante les parties de lèvre (22),
**caractérisé en ce que** les parties de lèvre (22) sont raccordées par le biais de deuxièmes articulations (22b) afin de former une boucle continue.

2. Paquet de fil de suture selon la revendication 1, **caractérisé en ce qu'**un support de flexion, en particulier une articulation vivante, est prévu en tant que première articulation (22a).

3. Paquet de fil de suture selon la revendication 1 ou 2, **caractérisé en ce que** la paroi supérieure (31) de l'élément de couvercle (3) est pourvue d'au moins un trou (36) de réception de broche d'ouverture de lèvre.

4. Paquet de fil de suture selon l'une quelconque des revendications 1 à 3, **caractérisé en ce qu'**une paroi de piste externe (27) faisant saillie vers la paroi supérieure (31) est disposée au niveau de l'extrémité externe de la partie de paroi périphérique (21).

5. Paquet de fil de suture selon la revendication 4, **caractérisé en ce qu'**un rebord supérieur de la paroi de piste externe (27) vient en butée contre la périphérie de la surface arrière de la paroi supérieure (31).

6. Paquet de fil de suture selon la revendication 4 ou 5, **caractérisé en ce que** la paroi de piste externe (27) et la paroi supérieure (31) sont pourvues de protubérances de verrouillage mutuel.

7. Paquet de fil de suture selon la revendication 6, **caractérisé en ce que** la paroi de piste externe et la paroi supérieure sont pourvues de dents (28) et de nervures (37a).

8. Procédé pour enrouler un fil de suture (4) dans un paquet de fil de suture (1), le paquet de fil de suture (1) comprenant un élément de base en forme de plateau (2) ayant une partie centrale (20) et une partie de paroi périphérique (21) et un élément de couvercle (3) configuré de sorte à être fixé à l'élément de base (2) et ayant une paroi de piste interne (30) et une paroi supérieure (31) s'étendant radialement en dehors de la paroi de piste interne (30) au niveau d'une extrémité supérieure de celle-ci, dans lequel un canal de fil de suture (10) est formé entre la paroi supérieure (31) et la partie de paroi périphérique (21), la partie de paroi périphérique (21) comprenant une pluralité de parties de lèvre (22) disposées de manière répartie sur la circonférence de la partie centrale (20) et une première articulation (22a) pour supporter de manière pivotante les parties de lèvre (22), et les parties de lèvre (22) étant connectées par le biais de deuxièmes articulations (22b) pour former une boucle continue, le procédé comprenant le fait de déformer élastiquement au moins en partie la partie de paroi périphérique (21) en faisant pivoter les parties de lèvre (22) autour d'un axe d'articulation pour écarter la partie de paroi périphérique (21) au moins en partie de la paroi supérieure (31).

9. Procédé selon la revendication 8, dans lequel la paroi supérieure (31) de l'élément de couvercle (3) est pourvue d'au moins un trou de réception de broche d'ouverture de lèvre (36) et dans lequel le procédé consiste à insérer au moins une broche d'ouverture de lèvre (60) dans le ou les trous de réception de broche d'ouverture de lèvre (36) pour écarter la partie de paroi périphérique (21) au moins en partie de la paroi supérieure (31).

10. Procédé selon l'une quelconque des revendications 8 et 9, dans lequel un doigt d'enroulement de fil (5) est inséré dans un espace entre la partie de paroi périphérique (21) et la paroi supérieure (31).

11. Procédé selon l'une quelconque des revendications 8 à 10, dans lequel le paquet de fil de suture (1) est tourné dans un plan parallèle à l'élément de base (3) pour enrouler le fil de suture (4) sur le paquet de fil de suture (1).
